# EUROPEAN PATENT SPECIFICATION

(11) **EP 3 331 434 B1**
(45) Date of publication and mention of the grant of the patent: **17.06.2020**
(21) Application number: 16750777.1
(22) Date of filing: 05.08.2016
(51) Int. Cl.: A61B 5/0476, A61N 5/06, A61N 1/372

(54) **METHOD AND DEVICE FOR MODULATING FEAR AND/OR ANXIETY**
VERFAHREN ZUR MODULATION VON ANGST UND/ODER ANGSTZUSTÄNDEN
PROCÉDÉ DE MODULATION DE LA PEUR ET/OU DE L' ANXIÉTÉ

(30) Priority: 06.08.2015 EP 15306274
(43) Date of publication of application: 13.06.2018
(73) Proprietor: Herry, Cyril, 33690 Cauvignac (FR); Dejean, Cyril, 33800 Bordeaux (FR)
(72) Inventor: HERRY, Cyril, 33077 Bordeaux (FR); DEJEAN, Cyril, 33077 Bordeaux (FR); COURTIN, Julien, 4058 Basel (CH); KARALIS, Nikolaos, 82152 Planegg-Martinsried (DE); PIAZZA, Pier-Vincenzo, 33077 Bordeaux (FR); MICHELET, Thomas, 33076 Bordeaux (FR)
(74) Representative: Plasseraud IP
(86) International application number: PCT/EP2016/068788
(87) International publication number: WO 2017/021542

(56) References cited:
- US-A1- 2013 245 714
- US-A1- 2014 155 952
- DEJEAN CYRIL ET AL: "Neuronal Circuits for Fear Expression and Recovery: Recent Advances and Potential Therapeutic Strategies", BIOLOGICAL PSYCHIATRY, ELSEVIER SCIENCE, NEW YORK, NY; US, vol. 78, no. 5, 24 March 2015 (2015-03-24) , pages 298-306, XP029247112, ISSN: 0006-3223, DOI: 10.1016/J.BIOPSYCH.2015.03.017
- JULIEN COURTIN ET AL: "Prefrontal parvalbumin interneurons shape neuronal activity to drive fear expression", NATURE, vol. 505, no. 7481, 20 November 2013 (2013-11-20), pages 92-96, XP055246988, United Kingdom ISSN: 0028-0836, DOI: 10.1038/nature12755 cited in the application
- JOSHUA H SIEGLE ET AL: "Enhancement of encoding and retrieval functions through theta phase-specific manipulation of hippocampus", E-LIFE, vol. 157, 1 January 2014 (2014-01-01), page 845, XP055247007, GB ISSN: 2050-084X, DOI: 10.1016/j.cell.2014.04.009
- COURTIN J ET AL: "Medial prefrontal cortex neuronal circuits in fear behavior", NEUROSCIENCE, NEW YORK, NY, US, vol. 240, 14 March 2013 (2013-03-14), pages 219-242, XP028585017, ISSN: 0306-4522, DOI: 10.1016/J.NEUROSCIENCE.2013.03.001

## Description

### Technical field

The present invention relates to the technical field of behaviour modulation in a subject, particularly a mammal, e.g. rodent and human. More particularly, the present invention relates to a method and a device for modulating fear and/or anxiety.

### Prior art

Fear is an unpleasant emotion caused by the threat of danger, pain or harm. Fear is a component of anxiety, which is an unpleasant feeling that involves a complex combination of emotions that include fear, apprehension, and worry. It is a psychological and physiological state that is often described as having cognitive, somatic, emotional, and behavioural components (1).

The cognitive component occasions expectation of a diffuse and uncertain danger. Somatically, the body prepares the organism to deal with threat (known as emergency reaction): blood pressure and heart rate are increased, sweating occurs and is exacerbated, blood flow to the major muscle groups is increased while immune and digestive system functions are inhibited. Externally, somatic signs of anxiety may include pale skin, sweating, trembling, and pupillary dilation. Emotionally, anxiety causes a sense of dread or panic and physically causes nausea, and chills. Behaviourally, both voluntary and involuntary behaviours may arise and are directed at escaping or avoiding the source of anxiety.

Anxiety is a pathological form of fear and will be understood as such within the present disclosure, whereas the word "fear" will be understood as the normal form (*i.e.* non-pathological) fear. Anxiety disorders may have acute or chronic manifestations. When sudden, the disorder is commonly referred to as acute anxiety. When gradual, the disorder can take hold over a period of several years (chronic), and may impair or prevent normal daily activities. As such, anxiety disorders are often debilitating chronic conditions, which can be present from an early age or begin suddenly after a triggering event. They are prone to flare up particularly at times of high stress.

Fear and anxiety can be parted in human by having the subject to follow a structured diagnostic interview adapted to the current diagnostic criteria proposed by the standard nosographical classifications of mental disorders of the Diagnostic and Statistical Manual of Mental Disorders, 5th edition (DSM-5). The main criterion to distinguish fear from anxiety is the observation of an exacerbated, disproportionate and non-adapted anxiety response with regard to a particular situation that should normally trigger a standard fear response.

Examples of fear comprise fear when facing an examination, an interview, a potential threat, an oral presentation, severe illness (cancer, pulmonary disease, heart disease....), chronic pain, and surgical procedure. Treatment of these kinds of fear is a non-therapeutic treatment since experiencing fear is not a medical condition.

Example of anxiety comprises but it is not limited to: generalised anxiety disorder, panic disorder, agoraphobia, other specific phobias, social anxiety disorder, obsessive-compulsive disorder, post-traumatic stress disorder, separation anxiety, exposure anxiety, anticipatory anxiety that induces sleep disorders, etc.

Generalised anxiety disorder is characterised by long-lasting anxiety that is not focused on any particular object or situation. People with generalised anxiety disorder feel afraid of something but are unable to articulate the specific fear. They fret constantly and have hard time controlling their worries. This is accompanied by muscle tension, difficulty to concentrate, irritability, restlessness and insomnia. These physical complaints, combined with the intense, long-term anxiety, make it difficult to cope with normal daily activities.

In panic disorder, a person suffers from brief attacks of intense terror and apprehension that cause trembling and shaking, confusion, dizziness, nausea, abdominal distress, difficulty breathing, chills or heat sensations, and feelings of impending doom or losing control, going crazy or fear of dying. The American Psychiatric Association (2000) defines a panic attack as fear or discomfort that arises abruptly and peaks in 10 minutes or less, and can occasionally last 20 to 30 min. Although panic attacks sometimes seem to occur out of nowhere, they generally happen after frightening experiences, prolonged stress, or even exercise. Panic disorders can be diagnosed when several apparently spontaneous attacks lead to a persistent concern about future attacks. Patients worry excessively and even quit jobs or refuse to leave home to avoid future attacks. In this context, agoraphobia is generally recognized as a common complication of panic disorder. The sufferer is anxious about being in a place or situation where escape is difficult or embarrassing (2, 3), or in which help may be difficult to be available in the event where unexpected or situational panic attack occurs suddenly.

Specific phobias involve strong, irrational fear and avoidance of a given object or situation that is the specific stimulus for this type of anxiety disorders. The persons suffering from phobias have especially powerful imaginations enabling them to anticipate vividly exposure to the object or situation what results in avoidance. The person is aware that the fear is excessive and irrational, yet they remain anxious and are unable to control their anxiety. Examples of specific phobias are phobias of animals, environmental situations (storms, water falls...), blood and surgeries, specific situations (bridges, elevators, travels...), suffocation and situations that may lead to suffocations, space, and children phobias related to loud sounds and disguise.

Social anxiety disorder is also known as social phobia. Individuals with this disorder experience intense fear of being negatively evaluated by others or of being publicly embarrassed or humiliated because of impulsive acts. Their fear of public scrutiny and potential humiliation or rejection becomes so pervasive that daily life can become markedly impaired and social interactions are significantly limited (4). Another social phobia is love-shyness, which most adversely affects certain men. Those afflicted find themselves unable to initiate intimate adult relationships (5).

Obsessive compulsive disorder (OCD) is a type of mental disorder primarily characterized by the combination of obsessions and compulsions. Obsessions are distressing, repetitive or persistent, intrusive, uncontrolled thoughts, images or impulsions that the individual often realizes are senseless. They classically generate severe anxiety, thereby leading to compulsions that are repetitive mental acts or behaviours that the person feels forced or compelled into doing, in order to neutralize or reduce anxiety and emotional distress.

Post-traumatic stress disorder (PTSD) is an anxiety disorder which results from a traumatic experience. The sufferer may experience flashbacks, severe anxiety until having a panic attack when exposed to stimuli or situations previously associated with the trauma, therefore leading to the emergence of avoidant behaviours. Symptoms that appear immediately after the trauma and last a maximum of 1 month are described as so-called acute stress disorder. The diagnostic of PTSD is positive if presence of the symptoms lasts for more than one month.

Separation Anxiety affects school aged children who struggle to engage socially or participate in the absence of their primary care giver. Separation anxiety can resemble school phobia.

Exposure anxiety consists of feeling one's own existence too extremely to withstand. Exposure anxiety was described as triggering a pervasive self-protective state of involuntary avoidance, diversion and retaliation responses resulting in a struggle to do things 'as oneself, 'by oneself or 'for oneself. By learning to do things as a 'non-self, i.e. taking on other characters, roles and voices, the individuals subjected to this disorder can sometimes still deal with the feeling.

Anticipatory anxiety is commonly observed in patients suffering from physchophysiological insomnia, which is characterized by heightened arousal and learned sleep-preventing associations that result in a complaint of insomnia. Patients suffering from this type of insomnia demonstrate excessive focus and anticipatory anxiety about sleep and suffer from elevated levels of cognitive and somatic arousal, particularly at bedtime.

There is converging evidence that anxiety disorders involve dysfunction of the amygdala and its interactions with the prefrontal cortex and the hippocampus (6). Classical fear conditioning is a powerful translational animal model widely used to study the neuronal substrates underlying anxiety disorders in humans and animals (7). In classical fear conditioning, pairing an initially neutral stimulus (conditioned stimulus; CS), with an aversive stimulus (unconditioned stimulus; US) leads to the formation of a robust and long-lasting fear memory (5, 6, 7). Whereas the formation of conditioned fear memories is known to depend on the integrity of the amygdala (8-10), there is now strong evidence that the medial prefrontal cortex plays a key role in the regulation of fear responses (10-12). Indeed, lesions or reversible inactivation of the dorsal medial prefrontal cortex (dmPFC) blocked conditioned fear responses (13, 14), whereas its electrical stimulation enhanced conditioned fear responses (15). Finally, neuronal activity in putative dmPFC excitatory pyramidal neurons was found to correlate with fear behaviour (16, 17). The dmPFC neuronal mechanisms allowing the control of conditioned fear responses are, however, not known.

Like any other cortices, the dmPFC contains around 80% of putative projection excitatory neurons and 20% of local inhibitory interneurons among which the vast majority correspond to interneurons expressing the calcium-binding protein parvalbumin (18). Pavalbumin-expressing (PV+) interneurons target the soma and axon hillock of excitatory projection neurons and precisely control their output activity (18). Moreover, PV+ interneurons are highly divergent and one single PV+ interneuron can contact and inhibit up to 1500 excitatory projection neurons (19). The optogenetic activation or inhibition of PV+ interneurons, which produces a strong inhibition or activation of excitatory projection neurons, has been shown to be causally related to fear inhibition and fear expression, respectively (11). Interestingly, conditioned fear behaviour in both humans and animal has been correlated with the development of prefrontal EEG oscillations in the 2 to 7 Hz range (20).

Conventional treatments for anxiety disorders include behavioural therapy, lifestyle changes and/or pharmaceutical therapy (medications). Most drugs used to treat these disorders are known to have negative side effects that may limit their use, or cause habituation and dependence. Moreover, even after short-term successful treatment, long-lasting recovery of symptoms is observed in most of the cases (21). Thus, there is still a need for an alternative method.

It has been contemplated to treat anxiety through electric stimulation applied unspecifically to the brain (US 2014/0155952). Other authors have voiced out the hypothesis that manipulating parvalbumin-expressing interneurons at specific phases of cortical oscillations may represent an additional strategy to rescue traumatic fear memories (22). Parvalbumin-expressing interneurons may be primarily found in the thalamus and hippocampus, but also anywhere in the brain. Further, the cortical oscillations are merely oscillations that may be register on the surface of the brain and are as such not specific to any region of the brain. Finally, the specific phases during which the parvalbumin-expressing interneurons were to be manipulated and how they were to be manipulated were at that time still to be determined.

DEJEAN CYRIL ET AL: "Neuronal Circuits for Fear Expression and Recovery: Recent Advances and Potential Therapeutic Strategies", BIOLOGICAL PSYCHIATRY, ELSEVIER SCIENCE, NY; US, vol. 78, no. 5, 24 March 2015, pages 298-306, shows a method for modulating fear in a healthy subject. JULIEN COURTIN ET AL: "Prefrontal parvalbumin interneurons shape neuronal activity to drive fear expression", NATURE,vol. 505, no. 7481, 20 November 2013, pages 92-96, shows inhibition/activation of parvalbumin-expressing interneurons to control fear expression.

JOSHUA H SIEGLE ET AL: "Enhancement of encoding and retrieval functions through theta phase-specific manipulation of hippocampus", E-LIFE,vol. 157, 2014, page 845, shows optogenetic stimulation triggered by theta waves.

### Summary of the invention

One objective of the invention is thus to overcome at least one drawback of the prior art. The present invention lies in the discovery that during an animal translational model of fear and/or anxiety, prefrontal excitatory neurons (i.e. neurons in the prefrontal cortex, more particularly in the medial prefrontal cortex, still more particularly in the dorsal part of the medial prefrontal cortex) are simultaneously activated during an ascending or descending phase of a periodic electric potential (oscillations) of neuronal origin that can be captured and processed into an electrophysiological signal. These prefrontal excitatory neurons, thus, form neuronal cell assemblies, that is a group of neurons simultaneously activated during short period of time. More precisely, the present inventions have discovered that decreasing or increasing the simultaneous activity of prefrontal excitatory neurons selectively at the ascending or descending phase of the oscillations, for example by respectively activating or inhibiting parvalbumin-expressing interneurons (PV+ In), successfully decrease or increase fear responses in a translational model of anxiety. The invention is defined by the claims.

The present disclosure provides a method for modulating fear and/or anxiety in a subject, for example fear in a healthy subject, the method comprising the steps of:
- acquiring an electrophysiological signal representative of the activity of at least a portion of the prefrontal cortex;
- selectively detecting ascending, respectively descending, phase of 2-7 Hz oscillations of the electrophysiological signal; and
- decreasing or increasing simultaneous activity of prefrontal excitatory neurons of the portion of prefrontal cortex selectively during the ascending, respectively descending, phase of the oscillations of the electrophysiological signal when an ascending, respectively descending, phase is detected.

Although it is known that decreasing or increasing simultaneous activity of prefrontal excitatory neurons of a portion of the prefrontal cortex, for example by activation or inhibition of PV+ interneurons, are causally related to fear inhibition and fear expression, respectively, and that conditioned fear behaviour in both humans and animal has been correlated with the development of prefrontal EEG oscillations in the 2 to 7 Hz range, it has still be unknown that the critical instant when physiological interference must be used onto the PV+ In was selectively the ascending or descending phase of a 2-7 Hz oscillations of the electrophysiological signal depending on the desired outcome and the desired way to reach the outcome. By doing so, all activation or inhibition occurrences are efficient. In other words it was unknown that influencing simultaneous activity of prefrontal excitatory neurons during the ascending phase or descending phase of the 2-7 Hz oscillations would have opposite effects on fear and anxiety. For example, stimulation of PV+ In in the ascending phase would decrease fear and anxiety, whilst their stimulation in the descending phase would increase fear and anxiety.

In one embodiment, decreasing or increasing simultaneous activity of prefrontal excitatory neurons is carried out by respectively activating or inhibiting PV+ In in the portion of the prefrontal cortex during the ascending, respectively descending, phase of the oscillations of the electrophysiological signal when an ascending phase is detected.

In one embodiment, PV+ In are activated or inhibited through one of the following:
- optogenetic stimulation;
- direct or transdermal electrical stimulation;
- transcranial magnetic stimulation;
- transcranial ultrasound;
- deep brain electrical stimulation
thus achieving decrease or increase respectively of fear and/or anxiety in a subject.

In one embodiment, acquiring the electrophysiological signal further comprises:
- capturing a raw signal from the portion of the prefrontal cortex;
- processing the raw signal to obtain the electrophysiological signal.

Processing the raw signal can further comprise digitalising the raw signal, preferably digitalising the raw signal comprises the following steps:
- preamplifying the raw signal;
- A/D converting the preamplified raw signal;
- amplifying the A/D converted raw signal.

In one embodiment, detecting ascending, respectively descending, phase further comprises 2-7 Hz filtering the electrophysiological signal through a 2-7 Hz filter.

In one embodiment, modulating fear and/or anxiety is decreasing fear and/or anxiety by detecting ascending phase of 2-7 Hz oscillations of the electrophysiological signal, and by decreasing simultaneous activity of prefrontal excitatory neurons of the portion of prefrontal cortex selectively during the ascending phase of the oscillations of the electrophysiological signal when an ascending phase is detected.

Alternatively, modulating fear and/or anxiety is increasing fear and/or anxiety by either:
detecting ascending phase of 2-7 Hz oscillations of the electrophysiological signal, and by increasing simultaneous activity of prefrontal excitatory neurons of the portion of prefrontal cortex selectively during the ascending phase of the oscillations of the electrophysiological signal when an ascending phase is detected; or
detecting descending phase of 2-7 Hz oscillations of the electrophysiological signal, and by decreasing simultaneous activity of prefrontal excitatory neurons of the portion of prefrontal cortex selectively during the descending phase of the oscillations of the electrophysiological signal when a descending phase is detected.

The disclosure also provides a device or a system for modulating fear and/or anxiety in a subject, comprising:
- a recorder for acquiring an electrophysiological signal representative of the activity of at least a portion of the prefrontal cortex;
- a phase detector for selectively detecting ascending or descending phase of 2-7 Hz oscillations of the electrophysiological signal;
- a modulator for decreasing or increasing simultaneous activity of prefrontal excitatory neurons of the portion of prefrontal cortex selectively during the ascending or descending phase of the oscillations of the electrophysiological signal when an ascending or descending phase is detected.

In one embodiment, the modulator is an activator or an inhibitor for activating or inhibiting PV+ In of at least a portion of the prefrontal cortex during the ascending or descending phase of the oscillations of the electrophysiological signal when an ascending or descending phase is detected.

In one embodiment, the device is an implantable device or a non-invasive device. In another embodiment, the system is partly implantable or non-invasive.

In one embodiment, the sensor further comprises:
- a sensor for capturing a raw signal from the portion of the prefrontal cortex;
- a processor for processing the raw signal to obtain the electrophysiological signal, preferably comprising a digitaliser for digitalising the raw signal into the electrophysiological signal;
preferably the digitaliser comprises:
- an amplifier for amplifying the raw signal;
- A/D convertor for A/D converting the amplified raw signal;

In one embodiment, the activator comprises a stimulator; preferably one of the following:
- optogenetic stimulator;
- direct or transdermal electrical stimulator;
- transcranial magnetic stimulator;
- transcranial ultrasound stimulator;
for achieving decrease of fear and/or anxiety when the stimulator activates the PV+ In.

The activator may further comprise a pulse generator for generating a pulse signal to the stimulator, when the phase detector detects an ascending phase of the 2-7 Hz oscillations of the electrophysiological signal.

The device or system may be used for decreasing anxiety, preferably one of generalised anxiety disorder, panic disorder, agoraphobia, specific phobias, social anxiety disorder, obsessive compulsive disorder, post-traumatic stress disorder, separation anxiety, exposure anxiety and anticipatory anxiety inducing sleep disorders;
or for increasing fear and/or anxiety in subjects with antisocial behaviour, preferably in sexual offenders, violent individuals in order to inhibit the unwanted behaviour.

Examples of fear and anxiety are given in the previous section.

### Drawings

Further objectives, advantages and features of the present invention will be apparent from the following description of exemplified embodiments together with the illustrating and nonlimiting accompanying drawings, wherein:
Figure 1 is a schematic representation of a device or system for modulating fear and/or anxiety in a subject according to the invention;
Figure 2 is a flow chart showing the steps of a method for modulating fear and/or anxiety in a subject according to the invention;
Figure 3 is a diagram showing freezing behaviour of wild type mice (black) and GFP mice (white) to CS+, baseline indicates freezing level when no CS+ is applied, CS+ indicates freezing level during CS+ presentations and Post-CS+ indicates freezing level during the 30 s following application of CS+;
Figure 4 is a diagram showing the freezing level of wild type mice (black) and GFP mice (white) during and after application of CS+, shaded window indicates moment at which optic stimulation is applied to verify whether optic stimulation has discrepant outcome on GFP mice in comparison to wild type mice;
Figure 5 is a diagram showing the freezing level of GFP mice (control) and ChR2 mice (activation of PV+ In) when optical stimulation is applied during ascending phase of the 2-7 Hz oscillations of the electrophysiological signals within a 30 s time window following application of CS+;
Figure 6 is a diagram showing the freezing level of GFP mice (control) and ArchT mice (inhibition of PV+ In) when optical stimulation is applied during ascending phase of the 2-7 Hz oscillations of the electrophysiological signals within a 30 s time window following application of CS+;
Figure 7 is a diagram showing the freezing level of GFP mice (control), ChR2 mice (activation of PV+ In) and ArchT mice (inhibition of PV+ In) when optical stimulation is applied during descending phase of the 2-7 Hz oscillations of the electrophysiological signals within a 30 s time window following application of CS+; and
Figure 8 is a diagram showing the freezing level of wild type mice depending on whether electric stimulation is applied or not during ascending phase of the 2-7 Hz oscillations of the electrophysiological signals within a 30 s time window following application of CS+.
Figure 9 is a diagram showing the pulse count as a function of phase of 3-6 Hz oscillations as used for stimulating PV+ In in ChR2 mice during ascending phase of 3-6 Hz oscillations.
Figure 10 is a diagram showing freezing behaviour in ChR2 mice during presentation of blocks of 4 CS+, while optogenetically stimulating ChR2 mice during ascending phase (dark) or descending phase (light) of 3-6 Hz oscillation 24 hr after conditioning (left) and without optogenetic stimulation 24 hr after optogenetic stimulation (right).
Figure 11 is a diagram showing freezing behaviour in ChR2 mice after presentation of blocks of 4 CS+, while optogenetically stimulating ChR2 mice during ascending phase (dark) or descending phase (light) of 3-6 Hz oscillation 24 hr after conditioning (left) and without optogenetic stimulation 24 hr after last optogenetic stimulation (right).

### Description of the invention

In the present disclosure, the word "fear" should be understood as the unpleasant emotion caused by the threat of danger, pain or harm, be the threat immediate or expected. As such, it encompasses the meaning of the word "anxiety", which is more commonly defined as a feeling of worry, nervousness, or unease about something with an uncertain outcome, or as a nervous disorder marked by excessive uneasiness and apprehension, typically with compulsive behaviour or panic attacks, but can have more complete and nuanced meanings as mentioned above.

"Modulating fear and/or anxiety" should be understood as meaning either decreasing the feeling of fear or anxiety down to complete suppression or increase of the feeling of fear or anxiety. Increasing the feeling of fear in a subject is particularly interesting for research purposes or as a therapy to repress unwanted behaviour comprising but not limited to sexual offenders, violent individual and any antisocial behaviour, whereas decreasing the feeling of fear or anxiety can be interesting for research or practical purposes such as helping an anxious person to feel less anxious and more generally treating anxiety related disorders including but not limited to generalised anxiety disorder, panic disorder, agoraphobia, other specific phobias (notably those listed above), social anxiety disorder, obsessive-compulsive disorder, post-traumatic stress disorder, separation anxiety, exposure anxiety, anticipatory anxiety inducing sleep disorders.

The word "subject" should be understood as meaning any living being that is capable of feeling fear or anxiety, preferably a mammal, still preferably chosen between a rodent, a primate, a dog or a cat. Among the rodents, rats, mice and rabbits may be mentioned. Among the primates, humans may be mentioned and more particularly either healthy humans and/or pathologically anxious humans.

The present invention provides both a device and a method for modulating fear and/or anxiety in a subject, which will be described hereafter in that order.

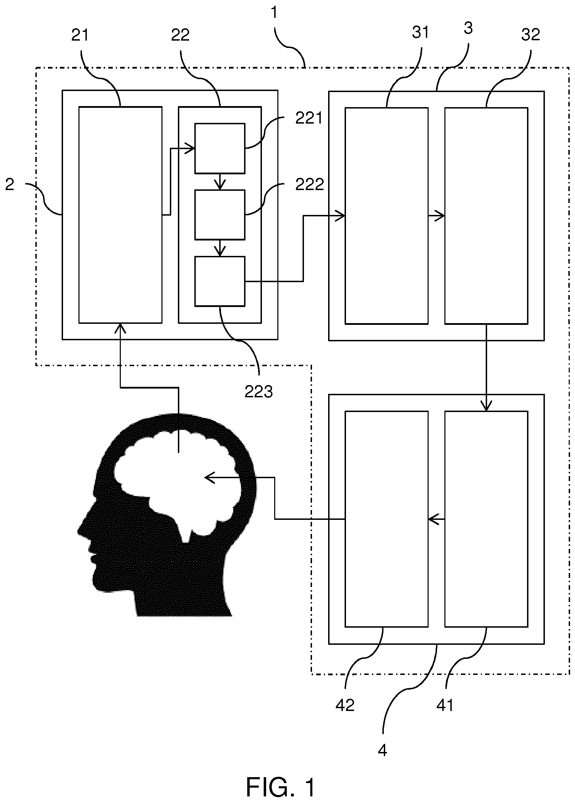

A device or system for modulating fear and/or anxiety in a subject according to the invention is described hereafter with reference to figure 1. It is to be understood that a "system" is comprised of a plurality of devices. Hereafter, only the word "device" will be used to ease and lighten the description but it should be understood as encompassing the meaning of system. As such, a given component of the device is to be understood as a corresponding device or a corresponding component of a device of the system that carries out the same functions as the given component of the device.

A device **1** for modulating fear and/or anxiety in a subject comprises a recorder **2,** a phase detector **3,** and a modulator **4.**

The recorder **2** is adapted to acquire an electrophysiological signal representative of the activity of at least a portion of the prefrontal cortex, preferably at least a portion of the medial prefrontal cortex, preferably at least a portion of the dorsal part of the prefrontal cortex.

The recorder **2** further comprises a sensor **21** and a processor **22.**

The sensor **21** is adapted to capture a raw signal from the corresponding portion of the prefrontal cortex and to send it to the processor **22.** The sensor **21** is more commonly at least one array of electrodes that are arranged to target the corresponding portion of the prefrontal cortex. Preferably the electrodes are orientable with respect to one another to finely tune the sensor **21** to the morphology of the subject.

The processor **22** is adapted to process the raw signal so that the electrophysiological signal is obtained and to send the electrophysiological signal to the phase detector **3.**

The processor **22** is preferably a digitaliser that digitalises the raw signal into the electrophysiological signal. Preferably the digitaliser comprises a preamplifier **221** for preamplifying the raw signal, an analogue/digital convertor (A/D convertor) **222** for receiving the preamplified raw signal and converting it into a A/D converted raw signal, and an amplifier **223** for receiving the A/D converted raw signal and amplifying it to the electrophysiological signal.

Examples of the recorder **2** are electroencephalography devices such as headstages, headsets, etc. among which: Enobio® 32 wearable electrophysiology sensor system of Neuroelectrics®; RHD2000-Series Evaluation System of Intan Technologies®; Neuro Prax TMS/tDC S EEG of Rogue-Resolution®; and EPOC® of Emotiv®.

The phase detector **3** is adapted to receive the electrophysiological signal from the recorder **2** and to detect selectively ascending or descending phases of 2-7 Hz oscillations, preferably 3-6 Hz oscillations, still preferably 4 Hz oscillations, of the electrophysiological signal. It is adapted to send an activation or inhibition signal to the activator or inhibitor **4** when it selectively detects an ascending or descending phases of 2-7 Hz oscillation, preferably 3-6 Hz oscillations, still preferably 4 Hz oscillations, of the electrophysiological signal.

It preferably comprises a 2-7 Hz filter **31** that receives the electrophysiological signal and 2-7 Hz band-pass-filters the electrophysiological signal into a filtered signal. Preferably the 2-7 Hz filter **31** is a 3-6 Hz filter **31,** still preferably 4 Hz filter **31** that receives the electrophysiological signal and specifically filters the electrophysiological signal into a filtered signal so that only 4 Hz signal is retained.

One example of a 2-7 Hz filter **31** is Power 1401-3 of Cambridge Electronic Design Ltd. (CED). Other examples are software filters such as Open Ephys (open source) and Matlab® of MathWorks®.

The phase detector **3** preferably also comprises a phase selector **32.** The phase selector **32** is able to select ascending or descending phases of 2-7 Hz waves, preferably 3-6 Hz waves, still preferably 4 Hz waves, comprised in the filtered signal. Preferably, the phase selector **32** sends the activation or inhibition signal when such a phase is detected.

Examples of a phase selector **32** are: R project (http://www.r-project.org), as Open Ephys (open source) and Matlab® of MathWorks®.

The modulator **4** is adapted to supress or promote the simultaneous activation of prefrontal excitatory neurons of the portion of prefrontal cortex during the ascending or descending phase of the 2-7 Hz oscillations, preferably 3-6 Hz oscillations, still preferably 4 Hz oscillations, of the electrophysiological signal when its ascending or descending phase is detected.

For example, the modulator is an activator or inhibitor **4** that is adapted to receive the activation or inhibition signal and to activate or inhibit respectively PV+ In in the corresponding portion of the prefrontal cortex, during the ascending or descending phase of the oscillations of the electrophysiological signal when the activation or inhibition signal is received.

Particularly, the activator **4** comprises a stimulator **42** that is preferably one of the following:
- optogenetic stimulator;
- direct or transdermal electrical stimulator;
- transcranial magnetic stimulator;
- transcranial ultrasound stimulator.
- deep brain electrical stimulation

Activating the PV+ In by stimulation during the ascending phase leads to decrease in fear and/or anxiety in the subject, whereas activating the PV+ In by stimulation during the descending phase or inhibiting the PV+ In by stimulation during the ascending phase leads to increase in fear and/or anxiety. It is obvious that if it is desired to act during the ascending phase, the components of the device **1** are set to act accordingly, i.e. detecting an ascending phase, stimulating during the ascending phase, *etc.* If it desired to act during the descending phase, the components of the device **1** are set to act according as well, i.e. detecting a descending phase, stimulating during the descending phase, *etc.*

The stimulator **42** typically comprises at least one array of electrodes that are arranged to target the corresponding portion of the prefrontal cortex. Preferably the electrodes are orientable with respect to one another to finely tune the stimulator **42** to the physiognomy of the subject.

In one embodiment, the at least one array of electrodes of the stimulator **42** and the at least one array of electrodes of the sensor **21** are one same array of electrodes. In another embodiment, they are distinct arrays of electrodes. In still another embodiment, parts of them are one same array and parts of them are distinct arrays.

Examples of stimulators are: Starstim (tDCS) of Neuroelectrics® (transdermal electrical stimulator); neuroConn (tDCS) of Rogue-Resolution® (transdermal electrical stimulator); DuoMAG™TMS (transcranial magnetic stimulator).

The activator **4** may further comprise a pulse generator **41** for receiving the activation signal and for generating a pulse signal and sending it to the stimulator **42,** when the activation signal is received.

One example of a pulse generator **41** is NI-USB 6211 of National Instrument®.

Preferably, the device **1** is a single implantable device or a single non-invasive device. The latter may be in the form of a helmet or cap to be worn onto or at the rear of the head of the subject. In such embodiment, all components described above are incorporated in the helmet or cap.

Alternatively, the device **1** is a partly implantable system. In other words, only part of the components described above is implantable, preferably as a single unit, such as at least the recorder **2,** optionally the activator or inhibitor **4** as well.

Alternatively still, the device **1** is a non-invasive system. In other words, all components described above are incorporated into distinct devices, such as for example:
- the recorder in a recording device, optionally the sensor in a sensing device and the processor in a processing device;
- the phase detector in a phase detecting device; and
- the activator or inhibitor in an activating or inhibiting device.

Other combinations are of course possible.

The device **1** can be used to help subject to overcome fear before or during a particularly stressful activity such as interview, examination, or any event linked with strong emotional load such as severe illness (cancer, pulmonary disease, heart disease....), chronic pain, and surgical procedure.

The device **1** can also be used in the treatment of anxiety disorders as classified in DSM-5, particularly generalised anxiety disorder, panic disorder, phobias such as agoraphobia, social anxiety disorder, obsessive-compulsive disorder, post-traumatic stress disorder, separation anxiety, exposure anxiety.

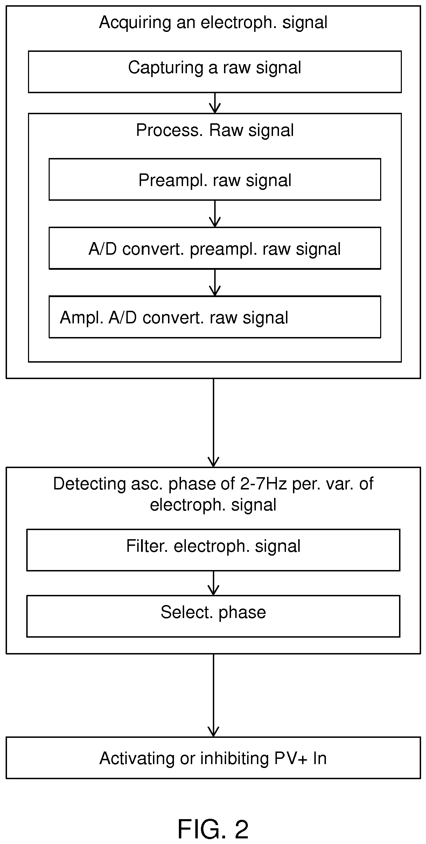

A method for modulating fear and/or anxiety is described hereafter with reference to figure 2.

The method comprises acquiring an electrophysiological signal representative of the activity of at least a portion of the prefrontal cortex, preferably at least a portion of the medial prefrontal cortex, preferably at least a portion of the dorsal part of the medial prefrontal cortex.

Acquiring the electrophysiological signal preferably comprises:
- capturing a raw signal from the corresponding portion of the prefrontal cortex;
- processing the raw signal to obtain the electrophysiological signal.

Indeed, raw signals are usually not usable as they are and need be processed, usually to provide signals that are easier to work with, such as amplified voltage signals and/or digital signal.

Therefore, processing the raw signal preferably comprises digitalising the raw signal. Digitalising the raw signal is preferably carried out as follows:
- preamplifying the raw signal;
- A/D converting the preamplified raw signal;
- amplifying the A/D converted raw signal.

Preamplifying the raw signal enables better signal-to-noise ratio and therefore better A/D converting.

The method further comprises detecting selectively ascending or descending phase of 2-7 Hz oscillations, preferably 3-6 Hz oscillations, still preferably 4 Hz oscillations, of the electrophysiological signal.

Detecting selectively ascending or descending phase preferably comprises 2-7 Hz, preferably 3-6 Hz, still preferably 4 Hz, filtering the electrophysiological signal through a 2-7 Hz filter, preferably 3-6 Hz filter, still preferably 4 Hz filter.

Filtering makes it possible to get rid of irrelevant elements of the electrophysiological signal.

Detecting selectively ascending or descending phase preferably comprises selecting a phase between 0° (the minima of the oscillation) and 180° (the peak of the oscillation). The selection is based on the calculation of the phase of the electrophysiological signal which is performed within a time window compatible with the period of the oscillation (2-7 Hz oscillations have a period comprised between 142 and 500 ms), preferably every 30 ms.

Detecting selectively ascending or descending phase preferably comprises a Hilbert transformation of the 2-7 Hz, preferably 3-6 Hz, still preferably 4 Hz, filtered electrophysiological signal (filtered signal) within a given time window, preferably of 500 ms, amplitude quantification of the filtered signal within the given time window, instantaneous phase retrieval of the filtered signal within the given time window, joint detection and sorting of signal bouts according to their amplitudes, and phase quantification for detecting the type of phase (ascending or descending). A low amplitude is an amplitude not significantly different from the amplitude recorded during baseline (2 min of recording before any stimulation), a high amplitude is an amplitude significantly higher from the amplitude recorded during baseline, preferably at least twice the amplitude of the average amplitude recorded during baseline, still preferably at least three times.

If the signal bout amplitudes are low, there is no simultaneous activity of the prefrontal excitatory neurons. If the signal bout amplitudes are high, simultaneous activity of the prefrontal excitatory neurons is detected.

The method further comprises decreasing or increasing simultaneous activity of prefrontal excitatory neurons of the portion of prefrontal cortex selectively during the ascending or descending phase of the oscillations of the electrophysiological signal when an ascending or descending phase is detected.

As mentioned above, decreasing or increasing simultaneous activity of prefrontal excitatory neurons enables fear and/or anxiety reduction or increase respectively. Fear and/or anxiety modulation is obtained by decreasing or increasing simultaneous activity of prefrontal excitatory neurons during the ascending or descending phase of the oscillations of the electrophysiological signal. Indeed, fear or anxiety episodes are characterised by prominent slow oscillations of between 2-7 Hz, preferably 3-6 Hz, still preferably at 4 Hz, in the prefrontal cortex, and more particularly in the dorsal part of the medial prefrontal cortex, that are observable through the electrophysiological signal. These slow oscillations have a sinusoidal shape. The ascending phase corresponds to the increase in amplitude of the electrophysiological signal during such slow oscillations. The descending phase corresponds to the decrease in amplitude of the electrophysiological signal during such slow oscillations.

For example, decreasing or increasing simultaneous activity of prefrontal excitatory neurons can be carried out through activating or inhibiting the PV+ In respectively during the ascending or descending phase of the oscillations of the electrophysiological signal when an ascending or descending phase is detected. Indeed, activation of PV+ In decreases the simultaneous activity of the prefrontal excitatory neurons, whereas inhibition of PV+ In favours the simultaneous activity of the prefrontal excitatory neurons.

Activating or inhibiting the PV+ In is preferably carried out through one of the following:
- optogenetic stimulation;
- direct or transdermal electrical stimulation;
- transcranial magnetic stimulation;
- transcranial ultrasonic stimulation.
- deep brain stimulation

Activating the PV+ In, or more generally, decreasing simultaneous activity of prefrontal excitatory neurons of the portion of prefrontal cortex, during the ascending phase of the oscillations of the electrophysiological signal triggers a decrease in fear or anxiety in the subject, whereas as activating the PV+ In, or more generally, decreasing simultaneous activity of prefrontal excitatory neurons of the portion of prefrontal cortex, during the descending phase of the oscillations of the electrophysiological signal or inhibiting the PV+ In, or more generally, increasing simultaneous activity of prefrontal excitatory neurons of the portion of prefrontal cortex, during the ascending phase of the oscillations of the electrophysiological signal triggers an increase in fear or anxiety in the subject. Activation or inhibition can be done through stimulation at a frequency equal or higher than 100 Hz. Indeed, PV+ In are the only cortex neurones that can follow discharges at a frequency of up to 1000 Hz. For example in rodents, an invasive electrical stimulation at 200 Hz only stimulates PV+ In while inhibits the other neurones. Optogenetic stimulation is preferably used in subject other than human and for research purposes. Indeed, optogenetic stimulation involves genetic manipulation to make the targeted cells (here the PV+ In) sensitive to light.

Direct electrical stimulation, as an invasive technique is also preferably used in subject for research purposes.

Non-invasive technique such as transdermal electrical stimulation, transcranial magnetic stimulation and transcranial ultrasonic stimulation are preferred because there are less likely to cause trauma in the subject.

The method, in particular when modulating fear and/or anxiety is decreasing fear and/or anxiety, can be used to help healthy subject to overcome fear before or during a particularly anxious activity such as interview, examination, or any event linked with strong emotional load.

The method, in particular when modulating fear and/or anxiety is decreasing fear and/or anxiety, can also be used in the treatment of anxiety disorders as classified in DSM-5, particularly generalised anxiety disorder, panic disorder, phobias such as agoraphobia and those mentioned above, social anxiety disorder, obsessive-compulsive disorder, post-traumatic stress disorder, separation anxiety, exposure anxiety.
the method, in particular when modulating fear and/or anxiety is increasing fear and/or anxiety, can be used for research purposes or as a therapy to repress unwanted behaviour comprising but not limited to sexual offenders, violent individual and any antisocial behaviour, etc.

### Methods

Optogenetic strategy based on the selective activation of PV+ In in the dorsal part of the medial prefrontal cortex (dmPFC) was used to demonstrate that formation of dmPFC neuronal assemblies is causally related to the expression of conditioned fear.

**Animals.** Male C57BL6/J mice (wild type, 3 months old, Janvier), Male PV-IRES-Cre mice (3 months old, Jackson Laboratory, B6;129P2-Pvalb^{tm1(cre)Arbr}/J) were individually housed for at least 7 days before all experiments, under a 12-h light-dark cycle, and provided with food and water *ad libitum.* All procedures were performed in accordance with standard ethical guidelines (European Communities Directive 86/60-EEC) and were approved by the committee on Animal Health and Care of Institut national de la santé et de la recherche médicale and French Ministry of Agriculture and Forestry (agreement #A3312001).

**Conditioning.** The mice were submitted to the conditioning protocol described as follows.

Fear conditioning and testing took place in two different contexts (contexts A and B). The conditioning and testing boxes were cleaned with 70% ethanol and 1% acetic acid before and after each session, respectively. To score freezing behaviour, a hallmark of fear responses in mice, an automated infrared beam detection system located on the bottom of the experimental chambers was used (Coulbourn Instruments). The animals were considered to be freezing if no movement was detected for 2 s.

On day 1, the mice were submitted to a habituation session in context A: they received four presentations of a first conditioned stimulus (CS+) and of a second conditioned stimulus (CS-). Total CS duration was 30 s and consisted of 27-time-repeated 50-ms pips at 0.9 Hz, 2 ms rise and fall. Pip frequency was 7.5 kHz or white-noise. Sound pressure level was 80 dB.

Discriminative fear conditioning was performed on the same day by pairing the CS+ with an unconditioned stimulus (US) consisting of 1 s foot-shock, 0.6 mA. 5 CS+/US pairings were performed with an inter-trial intervals of 20 to 180 s. The onset of the US coincided with the offset of the CS+. The CS- was presented after each CS+-US association but was never reinforced: five CS- presentations with inter-trial intervals of 20 to 180 s. The frequencies used for CS+ and CS- were counterbalanced across animals.

On day 2 and day 3, the mice were submitted to a post-fear-conditioning session in context: they received 4 presentations of the CS- followed by 4 presentations of the CS+. The number of CS+ was limited to 4 in order to prevent fear memory extinction from day 2 to day 3. Blue light stimulation was applied during 30 s following the last pip of the 4 CS+.

**Surgery and recordings of the electrophysiological signal.** Mice were anaesthetized with isoflurane (induction 3%, maintenance 1.5%) in O2. Body temperature was maintained at 37 °C with a temperature controller system (FHC). Mice were secured in a stereotaxic frame and unilaterally implanted in the left dorsomedial prefrontal cortex (dmPFC) with a multi-wire electrode array aimed at the following coordinates: 2 mm anterior to bregma; 0.3 mm lateral to the midline; and 0.8 to 1.4 mm ventral to the cortical surface. The electrodes consisted of 16 or 32 individually insulated nichrome wires (13 µm diameter, impedance 30-100 KΩ; Kanthal) contained in a 26-gauge stainless-steel guide cannula. The wires were attached to an 18-pin connector (Omnetics) or 2 connectors in the case of a 32 wire assembly. All implants were secured using Super-Bond cement (Sun Medical). After surgery mice were allowed to recover for 7 days and were habituated to handling. Analgesia was applied before, and 1 day after surgery (Metacam, Boehringer). Electrodes were connected to 1 or 2 headstages (Plexon) containing sixteen unity-gain operational amplifiers. Each headstage was connected to a 16-channel preamplifier (gain 100× bandpass filter from 150 Hz to 9 kHz for unit activity and from 0.7 Hz to 170 Hz for field potentials, Plexon). Spiking activity was digitized at 40 kHz and bandpass filtered from 250 Hz to 8 kHz, and isolated by time-amplitude window discrimination and template matching using a Multichannel Acquisition Processor system (Plexon). At the conclusion of the experiment, recording sites were marked with electrolytic lesions before perfusion, and electrode tips locations were reconstructed with standard histological techniques.

**Brainwave driven stimulation.** Neurons forming a functional assembly cannot be specifically manipulated using standard optogenetic approach. Although such cellular specificity is unattainable to this day, a certain temporal specificity exists as the activation of fear coding neuronal assemblies is specific of 2-7 Hz ascending phase. To apply our stimulation in a functionally specific manner we performed optogenetic inhibition of PNs as a function of the phase (ascending or descending) of the ongoing 2-7 Hz oscillation in dmPFC local field potentials (LFP). To that aim, a closed-loop stimulation protocol was designed, where animal behaviour (freezing) and dmPFC LFP (2-7 Hz power and phase) were monitored online and simultaneously analysed to drive a laser beam. To this aim, animal position was sampled at 80 Hz and LFP at 1 KHz then uploaded every 10 ms on Matlab for online analysis. The former consisted, on the one hand, in quantifying animal speed in the last 1000 ms of uploaded signal and on the other hand to band-pass filter the LFP signal (3 to 6 Hz range, a more drastic range than the actual 2-7 Hz) and retrieve both 3-6 Hz power in the last 500 ms and 3-6 Hz phase for the last recorded data point. Animal speed was calculated as the average distance travelled over the last 1000 ms time window and compared to the freezing threshold. Band-pass filtering was applied using a second order Butterworth filter and a Hilbert transform was subsequently used to estimate both 4 Hz power in the last 500 ms and LFP instantaneous phase for the last data point. Three conditions were defined for the stimulation. For the most recent data upload if:
(i) animal speed is below freezing threshold in the last 1000 ms,
(ii) 3-6 Hz power is above that of baseline level in the last 500 ms, and
(iii) 3-6 Hz phase is encompassed within the range of choice in the very last uploaded data point (ascending: 0 to 180° or descending 180° to 360°),
then stimulation was triggered. The former consisted in a 1 ms pulse sent from Matlab to a pulse generator that in turn sends a 30 ms pulse to a blue laser generator in order to deliver optical stimulation to the dmPFC through bilaterally implanted optic fibre. At the end of this 30 ms epoch if the 3 conditions are again fulfilled in the most recent 10 ms upload then a new stimulation pulse is triggered.

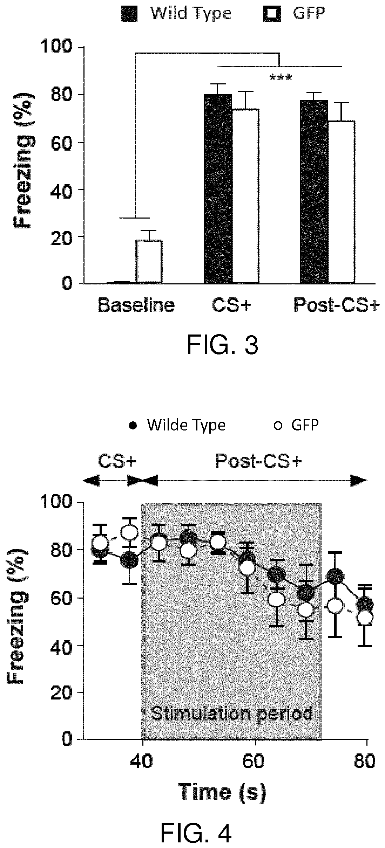

**Optogenetic strategy.** Male PV-IRES-Cre mice locally injected with conditional adeno-associated viruses (AAV) encoding for green fluorescent protein (GFP) were used as control (hereafter GFP mice). GFP mice and wild type mice show similar behaviour to CS+. Indeed, importantly freezing levels were of similar magnitude in GFP and wild type mice during CS+ and 30 s following CS+ (FIG. 3). Under optical stimulation during a time window of 30 s following CS+, freezing levels in GFP and wild type mice are also similar (FIG. 4). Thus, GFP mice can be used as control mice in optogenetic tests.

For activating or inhibiting PV+ In during the optogenetic tests, Male PV-IRES-Cre mice were locally injected with conditional AAV encoding for channelrhodopsin (activation, hereafter ChR2 mice) or archaerhodopsin (inhibition, hereafter ArchT mice) in the dmPFC.

In order to evaluate the changes in freezing levels upon optogenetic stimulation and under the assumption that ascending and descending phase stimulation respectively reduce and enhance fear response, ascending phase stimulation was tested on day 2 and descending phase on day 3 in order to avoid floor and ceiling effect respectively. Optical stimulation for activating ChR2 or ArchT proteins was applied after CS+. Analyses were restricted to the 30 s epoch following CS+ both on day 2 and day 3. Note that within these 30 s, light application was conditioned to the behaviour and the brain state of the animal (see Brainwave driven stimulation section above).

To score freezing behaviour, the aforementioned automated infrared beam detection was used in addition to a Cineplex video tracking system (Plexon) that was used for online detection of freezing and subsequent conditional optogenetic stimulation.

Levels of freezing for all mice were similar during CS+.

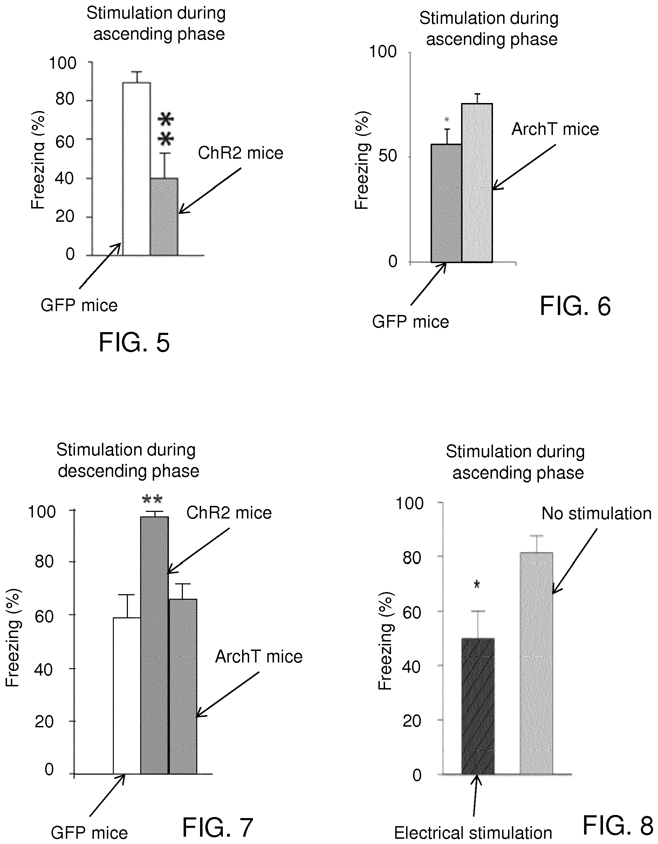

Optogenetic-mediated inhibition of the firing activity of dmPFC PNs by optical stimulation of ChR2 mice specifically during the ascending phase of the 2-7 Hz oscillations or the electrophysiological signals during freezing episodes significantly reduced freezing behaviour in comparison to control conditions (ChR2 mice: 40.26 ± 10.7 %; GFP mice: 88.1 ± 6.0 %) (FIG. 5).

Optogenetic-mediated inhibition of the firing activity of dmPFC PNs by optical sitmulation of ChR2 mice during the descending phase of the oscillation during freezing episodes, when dmPFC PNs participating to neuronal assemblies are less likely to be recruited, interestingly, significantly increased freezing behaviour in comparison to control conditions. This indicates that the sequential activation and inhibition of neuronal assemblies within 4 Hz oscillations strongly contribute to freezing behaviour (ChR2 mice: 97.3 ± 2.2 %; GFP mice: 58.7 ± 9.1 %) (FIG. 7).

Optogenetic-mediated activation of dmPFC PNs through the inhibition of dmPFC PV+ In by optical stimulation of ArchT mice during the ascending phase of the 2-7 Hz oscillations of the electrophysiological signal during freezing episodes increased freezing behaviour in comparison to control conditions (ArchT mice: 76 ± 3.2 % GFP mice: 56.3 ± 6.1 %) (FIG. 6).

Optogenetic-mediated activation of dmPFC PNs through the inhibition of dmPFC PV+ In by optical stimulation of ArchT mice during the descending phase of the oscillation had no effect on freezing behaviour further indicating the phase specificity of our manipulation (ArchT mice: 66.1 ± 7.7%; GFP mice: 58.7 ± 9.1 %) (FIG. 7).

**Electrical stimulation.** Analyses were restricted to the 30 s epoch following CS+ on day 1 after conditioning. Note that within these 30 s, electrical stimulation was conditioned to the behaviour and the brain state of the animal (same principle that the Brainwave driven stimulation section above, except that electrical and not optogenetic stimulation was used).

A first group of wild type mice were subjected to CS+ and to electric stimulation after CS+. Electric stimulation was carried out at a frequency of 200 Hz, at which value only PV+ In can follows the discharge frequency, and only during ascending phase of the 2-7 Hz oscillations of the electrophysiological signal. A second group of wild type mice was only subjected to CS+ and not to electric stimulation after CS+ and is used as control. Freezing level of both groups was recorded during the 30 s following CS+. As FIG. 8 shows, freezing level in wild type mice subjected to electric stimulation is significantly lower than that of wild type mice not subjected to electric stimulation.

**Long-lasting effect of stimulation during ascending phase of 3-6 Hz oscillations.** The mice as selected and conditioned as described above, except for day 2 and day 3. On day 2 and day 3, the mice were submitted to a post-fear-conditioning session in context. On day 2 they received 4 presentations of the CS- followed by 12 presentations of the CS+, and on day 3 they received 4 presentations of the CS- followed by 4 presentations of the CS+. Blue light stimulation was applied during each CS+ and during the 30 s following the last pip of the CS+ only during day 2. Brainwave driven stimulation was carried out as described above but animal behaviour (freezing) was no longer monitored and analysed, thus making sampling of animal position and quantifying animal speed unnecessary. Accordingly, condition (i) was dropped. For the optogenetic strategy, male PV-IRES-Cre mice were injected in the dmPFC with conditional adeno-associated viruses (AAV) encoding for channelrhodopsin (ChR2). To evaluate the long-term effect of ascending phase stimulation on fear behaviour, ascending phase stimulation was performed on day 2 and fear memory tested on day 3 without stimulation. Optical stimulation for activating ChR2 proteins in the ascending phase of the stimulation was applied during and 30 sec after each CS+. Analyses were performed during the CS+ presentations and during the 30 s epoch following CS+ both on day 2 and day 3. The long-term effect of ascending phase stimulation on fear behaviour was compared to the effect of descending phase stimulation.

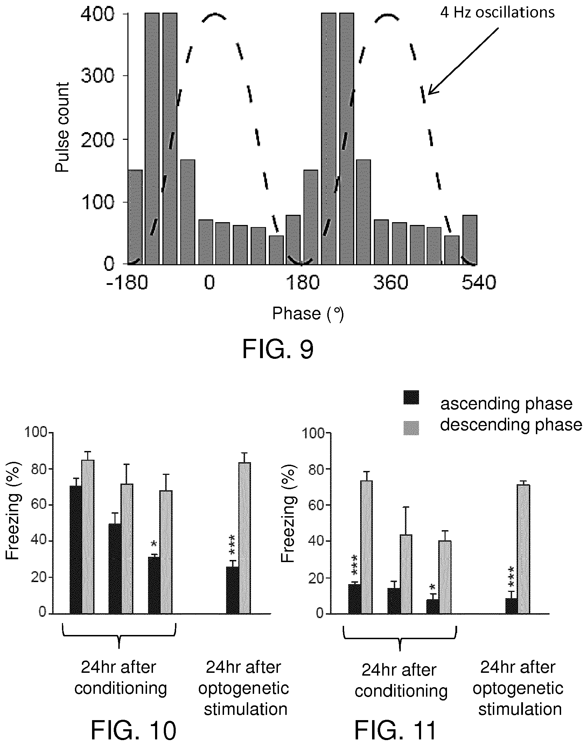

It was verified that success of specifically stimulating during ascending phases of 3-6 Hz oscillations was independent from freezing behaviour of the mice. Figure 9 shows a histogram of phase-pulse stimulation used for Figures 10 and 11 as well as detected 3-6 Hz oscillations, demonstrating that it was possible to specifically and accurately target ascending phases of 3-6 Hz oscillations. Further, it was demonstrated that the stimulation paradigm was achieved independently of the mice's freezing behaviour. Figure 10 shows that stimulation during the ascending phase results in a significantly decreased of freezing behaviour during CS+ presentation thus demonstrating that it facilitates fear memory extinction in comparison with stimulation during the descending phase. The striking difference is still to be noticed 24 hr later after the last optogenetic stimulation when the mice were presented with CS+. Figure 11 show that the same conclusion may be drawn with regards to freezing behaviour after CS+ presentation.

### Bibliography

1. Seligman MEP, Walker E, Rosenhan DL (2001): Abnormal psychology (4th ed.) New York: W.W. Norton.
2. Craske MG (2000): Panic Disorder. In. A.E. Kazdin (Ed.), Encyclopedia of Psychology (pp. 33-35). Washington, DC: American Psychological Association and New York, NY: Oxford University Press.
3. Gorman JM, Kent JM, Sullivan GM, Coplan JD (2000): Neuroanatomical hypothesis of panic disorder, revised. The American journal of psychiatry. 157:493-505.
4. den Boer JA (2000): Social anxiety disorder/social phobia: epidemiology, diagnosis, neurobiology, and treatment. Comprehensive psychiatry. 41:405-415.
5. Gilmartin BG (1987): Peer group antecedents of severe love-shyness in males. Journal of personality. 55:467-489.
6. Drevets WC, Price JL, Furey ML (2008): Brain structural and functional abnormalities in mood disorders: implications for neurocircuitry models of depression. Brain structure & function. 213:93-118.
7. Phelps EA, LeDoux JE (2005): Contributions of the amygdala to emotion processing: from animal models to human behavior. Neuron. 48:175-187.
8. LeDoux JE (2000): Emotion circuits in the brain. Annu Rev Neurosci. 23:155-184.
9. Maren S (2001): Neurobiology of Pavlovian fear conditioning. Annu Rev Neurosci. 24:897-931.
10. Maren S, Quirk GJ (2004): Neuronal signalling of fear memory. Nat Rev Neurosci. 5:844-852.
11. Courtin J, Chaudun F, Rozeske RR, Karalis N, Gonzalez-Campo C, Wurtz H, et al. (2014): Prefrontal parvalbumin interneurons shape neuronal activity to drive fear expression. Nature. 505:92-96.
12. Herry C, Johansen JP (2014): Encoding of fear learning and memory in distributed neuronal circuits. Nat Neurosci. 17:1644-1654.
13. Sierra-Mercado D, Jr., Corcoran KA, Lebron-Milad K, Quirk GJ (2006): Inactivation of the ventromedial prefrontal cortex reduces expression of conditioned fear and impairs subsequent recall of extinction. Eur J Neurosci. 24:1751-1758.
14. Corcoran KA, Quirk GJ (2007): Activity in prelimbic cortex is necessary for the expression of learned, but not innate, fears. The Journal of neuroscience : the official journal of the Society for Neuroscience. 27:840-844.
15. Vidal-Gonzalez I, Vidal-Gonzalez B, Rauch SL, Quirk GJ (2006): Microstimulation reveals opposing influences of prelimbic and infralimbic cortex on the expression of conditioned fear. Learn Mem. 13:728-733.
16. Burgos-Robles A, Vidal-Gonzalez I, Quirk GJ (2009): Sustained conditioned responses in prelimbic prefrontal neurons are correlated with fear expression and extinction failure. The Journal of neuroscience : the official journal of the Society for Neuroscience. 29:8474-8482.
17. Chang CH, Berke JD, Maren S (2010): Single-unit activity in the medial prefrontal cortex during immediate and delayed extinction of fear in rats. PLoS One. 5:e11971.
18. Markram H, Toledo-Rodriguez M, Wang Y, Gupta A, Silberberg G, Wu C (2004): Interneurons of the neocortical inhibitory system. Nat Rev Neurosci. 5:793-807.
19. Sik A, Penttonen M, Ylinen A, Buzsaki G (1995): Hippocampal CA1 interneurons: an in vivo intracellular labeling study. The Journal of neuroscience : the official journal of the Society for Neuroscience. 15:6651-6665.
20. Mueller EM, Panitz C, Hermann C, Pizzagalli DA (2014): Prefrontal oscillations during recall of conditioned and extinguished fear in humans. The Journal of neuroscience : the official journal of the Society for Neuroscience. 34:7059-7066.
21. Rodriguez BI, Craske MG, Mineka S, Hladek D (1999): Context-specificity of relapse: effects of therapist and environmental context on return of fear. Behaviour research and therapy. 37:845-862.
22. Dejean C, et al. (2015): Neuronal circuits for fear expression and recovery: Recent advances and potential therapeutic strategies. Society of Biological Psychiatry. 78:298-306

## Claims

1. A non-therapeutic method for modulating fear in a healthy subject comprising the steps of:
- acquiring an electrophysiological signal representative of the activity of at least a portion of the prefrontal cortex;
- selectively detecting ascending, respectively descending, phase of 2-7 Hz oscillations of the electrophysiological signal; and
- activating or inhibiting parvalbumin-expressing interneurons, PV+ In, of the portion of prefrontal cortex selectively during the ascending, respectively descending, phase of the oscillations of the electrophysiological signal when an ascending, respectively descending, phase is detected;
wherein when modulating fear is decreasing fear, decreasing fear is obtained by detecting ascending phase of 2-7 Hz oscillations of the electrophysiological signal, and by activating PV+ In of the portion of prefrontal cortex selectively during the ascending phase of the oscillations of the electrophysiological signal when an ascending phase is detected;
wherein when modulating fear is increasing fear, increasing fear is obtained by either:
detecting ascending phase of 2-7 Hz oscillations of the electrophysiological signal, and by inhibiting PV+ In of the portion of prefrontal cortex selectively during the ascending phase of the oscillations of the electrophysiological signal when an ascending phase is detected; or
detecting descending phase of 2-7 Hz oscillations of the electrophysiological signal, and by activating PV+ In of the portion of prefrontal cortex selectively during the descending phase of the oscillations of the electrophysiological signal when a descending phase is detected.

2. The method of claim 1, wherein PV+ In are activated or inhibited through one of the following:
- optogenetic stimulation;
- direct or transdermal electrical stimulation;
- transcranial magnetic stimulation;
- transcranial ultrasound;
- deep brain stimulation
thus achieving decrease or increase respectively of fear the healthy subject.

3. The method of any claims 1 to 2, wherein acquiring the electrophysiological signal further comprises:
- capturing a raw signal from the portion of the prefrontal cortex;
- processing the raw signal to obtain the electrophysiological signal.

4. The method of claim 3, wherein processing the raw signal comprises digitalising the raw signal, preferably digitalising the raw signal comprises the following steps:
- preamplifying the raw signal;
- A/D converting the preamplified raw signal;
- amplifying the A/D converted raw signal.

5. The method of any claim 1 to 4, wherein detecting ascending, respectively descending, phase further comprises 2-7 Hz filtering the electrophysiological signal through a 2-7 Hz filter.

6. A fear-decreasing device comprising:
- a recorder configured to acquire an electrophysiological signal representative of the activity of at least a portion of the prefrontal cortex;
- a phase detector configured to selectively detect ascending phase of 2-7 Hz oscillations of the electrophysiological signal;
- a modulator configured to activate PV+ In of the portion of prefrontal cortex selectively during the ascending phase of the oscillations of the electrophysiological signal when an ascending phase is detected.

7. A fear-increasing device comprising:
- a recorder configured to acquire an electrophysiological signal representative of the activity of at least a portion of the prefrontal cortex;
- a phase detector configured to selectively detect ascending or descending phase of 2-7 Hz oscillations of the electrophysiological signal;
- a modulator configured to inhibit PV+ In of the portion of prefrontal cortex selectively during the ascending phase of the oscillations of the electrophysiological signal when an ascending phase is detected, or to activate PV+ In of the portion of prefrontal cortex selectively during the descending phase of the oscillations of the electrophysiological signal when a descending phase is detected.

8. The device of any claim 6 to 7, wherein the device or system is one of an implantable device, a non-invasive device or system, partly implantable system.

9. The device of any claim 6 to 8, wherein the sensor further comprises:
- a sensor configured to capture a raw signal from the portion of the prefrontal cortex;
- a processor configured to process the raw signal to obtain the electrophysiological signal, preferably comprising a digitaliser for digitalising the raw signal into the electrophysiological signal;
preferably the digitaliser comprises:
- an amplifier configured to amplify the raw signal;
- A/D convertor configure to A/D convert the amplified raw signal;

10. The device of any claim 6 to 9, wherein the activator comprises a stimulator; preferably one of the following:
- optogenetic stimulator;
- direct or transdermal electrical stimulator;
- transcranial magnetic stimulator;
- transcranial ultrasound stimulator;
for achieving decrease or increase of fear and/or anxiety when the stimulator activates the PV+ In.

11. The device or system of claim 10, wherein the activator further comprises a pulse generator for generating a pulse signal to the stimulator, when the phase detector detects an ascending phase of the 2-7 Hz oscillations of the electrophysiological signal or when the phase detector detects a descending phase of the 2-7 Hz oscillations of the electrophysiological signal.

## Patentansprüche

1. Nicht-therapeutisches Verfahren zur Modulation von Furcht bei einem gesunden Individuum, umfassend die Schritte:
- Erfassen eines elektrophysiologischen Signals, das für die Aktivität mindestens eines Teils des präfrontalen Kortex repräsentativ ist;
- selektives Detektieren einer ansteigenden bzw. absteigenden Phase von 2-7 Hz-Oszillationen des elektrophysiologischen Signals; und
- selektives Aktivieren oder Inhibieren von Parvalbumin-exprimierenden Interneuronen, PV+ In, des Teils des präfrontalen Kortex während der ansteigenden bzw. absteigenden Phase der Oszillationen des elektrophysiologischen Signals, sobald eine ansteigende bzw. absteigende Phase detektiert wird;
wobei, sobald modulierte Furcht abnehmende Furcht ist, abnehmende Furcht durch Detektieren einer ansteigenden Phase von 2-7 Hz Oszillationen des elektrophysiologischen Signals und durch selektives Aktivieren von PV+ In des Teils des präfrontalen Kortex während der ansteigenden Phase der Oszillationen des elektrophysiologischen Signals, sobald eine ansteigende Phase detektiert wird, erhalten wird;
wobei, sobald modulierte Furcht zunehmende Furcht ist, zunehmende Furcht entweder durch Detektieren einer ansteigenden Phase von 2-7 Hz Oszillationen des elektrophysiologischen Signals und durch selektives Inhibieren von PV+ In des Teils des präfrontalen Kortex während der ansteigenden Phase der Oszillationen des elektrophysiologischen Signals, sobald eine ansteigende Phase detektiert wird; oder durch
Detektieren einer absteigenden Phase von 2-7 Hz Oszillationen des elektrophysiologischen Signals und durch selektives Aktivieren von PV+ In des Teils des präfrontalen Kortex während der absteigenden Phase der Oszillationen des elektrophysiologischen Signals, sobald eine absteigende Phase detektiert wird, erhalten wird.

2. Verfahren nach Anspruch 1, wobei PV+ In durch eines der folgenden aktiviert oder inhibiert werden:
- optogenetische Stimulation;
- direkte oder transdermale elektrische Stimulation;
- transkranielle Magnetstimulation;
- transkranieller Ultraschall;
- Tiefenhirnstimulation,
wodurch die Furcht des gesunden Individuums abnimmt bzw. zunimmt.

3. Verfahren nach einem der Ansprüche 1 oder 2, wobei Erfassen des elektrophysiologischen Signals ferner umfasst:
- Erfassen eines Rohsignals von dem Teil des präfrontalen Kortex;
- Verarbeiten des Rohsignals, um das elektrophysiologische Signal zu erhalten.

4. Verfahren nach Anspruch 3, wobei Verarbeiten des Rohsignals Digitalisieren des Rohsignals umfasst, wobei vorzugsweise das Digitalisieren des Rohsignals die folgenden Schritte umfasst:
- Vorverstärken des Rohsignals;
- A/D-Konvertieren des vorverstärkten Rohsignals;
- Verstärken des A/D-konvertierten Rohsignals.

5. Verfahren nach einem der Ansprüche 1 bis 4, wobei Detektieren einer ansteigenden bzw. absteigenden Phase ferner 2-7 Hz Filtern des elektrophysiologischen Signals durch einen 2-7 Hz Filter umfasst.

6. Vorrichtung zur Verringerung von Furcht, umfassend:
- einen Rekorder, ausgebildet, um ein elektrophysiologisches Signal, das für die Aktivität mindestens eines Teils des präfrontalen Kortex repräsentativ ist, zu erfassen;
- einen Phasendetektor, ausgebildet, um selektiv eine ansteigende Phase von 2-7 Hz Oszillationen des elektrophysiologischen Signals zu detektieren;
- einen Modulator, ausgebildet, um PV+ In des Teils des präfrontalen Kortex selektiv während der ansteigenden Phase der Oszillationen des elektrophysiologischen Signals, sobald eine ansteigende Phase detektiert wird, zu aktivieren.

7. Vorrichtung zur Verringerung von Furcht, umfassend:
- einen Rekorder, ausgebildet, um ein elektrophysiologisches Signal, das für die Aktivität mindestens eines Teils des präfrontalen Kortex repräsentativ ist, zu erfassen;
- einen Phasendetektor, ausgebildet, um selektiv eine ansteigende oder absteigende Phase von 2-7 Hz Oszillationen des elektrophysiologischen Signals zu detektieren;
- einen Modulator, ausgebildet, um PV+ In des Teils des präfrontalen Kortex selektiv während der ansteigenden Phase der Oszillationen des elektrophysiologischen Signals, sobald eine ansteigende Phase detektiert wird, zu inhibieren, oder um PV+ In des Teils des präfrontalen Kortex selektiv während der absteigenden Phase der Oszillationen des elektrophysiologischen Signals, sobald eine absteigende Phase detektiert wird, zu aktivieren.

8. Vorrichtung nach einem der Ansprüche 6 bis 7, wobei die Vorrichtung oder das System eines/einer einer implantierbaren Vorrichtung, einer nicht-invasiven Vorrichtung oder eines nicht-invasiven Systems, eines teilweise implantierbaren Systems ist.

9. Vorrichtung nach einem der Ansprüche 6 bis 8, wobei der Sensor ferner umfasst:
- einen Sensor, ausgebildet, um ein Rohsignal des Teils des präfrontalen Kortex zu erfassen;
- einen Prozessor, ausgebildet, um das Rohsignal zu verarbeiten, um das elektrophysiologische Signal zu erhalten, vorzugsweise umfassend einen Digitalisierer zum Digitalisieren des Rohsignals in das elektrophysiologische Signal;
vorzugsweise umfasst der Digitalisierer:
- einen Verstärker, ausgebildet um das Rohsignal zu verstärken;
- einen A/D-Konvertierer, um das verstärkte Rohsignal zu A/D-konvertieren;

10. Vorrichtung nach einem der Ansprüche 6 bis 9, wobei der Aktivator einen Stimulator umfasst, vorzugsweise einen der folgenden:
- optogenetischer Stimulator;
- direkter oder transdermaler elektrischer Stimulator;
- transkranieller Magnetstimulator;
- transkranieller Ultraschallstimulator;
zum Erreichen von Abnahme oder Zunahme von Furcht und/oder Angst, sobald der Stimulator die PV+ In aktiviert.

11. Vorrichtung oder System nach Anspruch 10, wobei der Aktivator ferner einen Pulsgenerator zur Generierung eines Pulssignals zu dem Stimulator, sobald der Phasendetektor eine ansteigende Phase der 2-7 Hz Oszillationen des elektrophysiologischen Signals detektiert oder sobald der Phasendetektor eine abnehmende Phase der 2-7 Hz Oszillationen des elektrophysiologischen Signals detektiert, umfasst.

## Revendications

1. Procédé non thérapeutique de modulation de la peur chez un sujet sain comprenant les étapes suivantes :
- acquisition d'un signal électrophysiologique représentatif de l'activité d'au moins une partie du cortex préfrontal ;
- détection sélective de la phase ascendante, respectivement descendante, d'oscillations de 2 à 7 Hz du signal électrophysiologique ; et
- activation ou inhibition d'interneurones exprimant la parvalbumine, PV+ In, de la partie du cortex préfrontal sélectivement durant la phase ascendante, respectivement descendante, des oscillations du signal électrophysiologique lorsqu'une phase ascendante, respectivement descente, est détectée ;
dans lequel lorsque la modulation de la peur est la réduction de la peur, la réduction de la peur est obtenue par détection de la phase ascendante d'oscillations de 2 à 7 Hz du signal électrophysiologique, et par activation des PV+ In de la partie du cortex préfrontal sélectivement durant la phase ascendante des oscillations du signal électrophysiologique lorsqu'une phase ascendante est détectée ;
dans lequel lorsque la modulation de la peur est l'augmentation de la peur, l'augmentation de la peur est obtenue par :
détection de la phase ascendante d'oscillations de 2 à 7 Hz du signal électrophysiologique, et par inhibition des PV+ In de la partie du cortex préfrontal sélectivement durant la phase ascendante des oscillations du signal électrophysiologique lorsqu'une phase ascendante est détectée ; ou
détection de la phase descendante d'oscillations de 2 à 7 Hz du signal électrophysiologique, et par activation des PV+ In de la partie du cortex préfrontal sélectivement durant la phase descendante des oscillations du signal électrophysiologique lorsqu'une phase descendante est détectée.

2. Procédé selon la revendication 1, dans lequel les PV+ In sont activés ou inhibés par l'un de :
- la stimulation optogénétique ;
- la stimulation électrique directe ou transdermique ;
- la stimulation magnétique transcrânienne ;
- les ultrasons transcrâniens ;
- la stimulation cérébrale profonde
permettant ainsi d'atteindre une réduction ou une augmentation respectivement de la peur chez le sujet sain.

3. Procédé selon l'une quelconque des revendications 1 à 2, dans lequel l'acquisition du signal électrophysiologique comprend en outre :
- la capture d'un signal brut de la partie du cortex préfrontal ;
- le traitement du signal brut pour obtenir le signal électrophysiologique.

4. Procédé selon la revendication 3, dans lequel le traitement du signal brut comprend la numérisation du signal brut, de préférence la numérisation du signal brut comprend les étapes suivantes :
- préamplification du signal brut ;
- conversion A/N du signal brut préamplifié ;
- amplification du signal brut ayant subi une conversion A/N.

5. Procédé selon l'une quelconque des revendications 1 à 4, dans lequel la détection de la phase ascendante, respectivement descendante, comprend en outre la filtration à 2 à 7 Hz du signal électrophysiologique à travers un filtre à 2 à 7 Hz.

6. Dispositif de réduction de la peur comprenant :
- un enregistreur configuré pour acquérir un signal électrophysiologique représentatif de l'activité d'au moins une partie du cortex préfrontal ;
- un détecteur de phase configuré pour sélectivement détecter la phase ascendante d'oscillations à 2 à 7 Hz du signal électrophysiologique ;
- un modulateur configuré pour activer les PV+ In de la partie du cortex préfrontal sélectivement durant la phase ascendante des oscillations du signal électrophysiologique lorsqu'une phase ascendante est détectée.

7. Dispositif d'augmentation de la peur comprenant :
- un enregistreur configuré pour acquérir un signal électrophysiologique représentatif de l'activité d'au moins une partie du cortex préfrontal ;
- un détecteur de phase configuré pour sélectivement détecter la phase ascendante ou descendante d'oscillations à 2 à 7 Hz du signal électrophysiologique ;
- un modulateur configuré pour inhiber les PV+ In de la partie du cortex préfrontal sélectivement durant la phase ascendante des oscillations du signal électrophysiologique lorsqu'une phase ascendante est détectée, ou pour activer les PV+ In de la partie du cortex préfrontal sélectivement durant la phase descendante des oscillations du signal électrophysiologique lorsqu'une phase descendante est détectée.

8. Dispositif selon l'une quelconque des revendications 6 à 7, dans lequel le dispositif ou système est l'un d'un dispositif implantable, d'un dispositif ou système non invasif, d'un système partiellement implantable.

9. Dispositif selon l'une quelconque des revendications 6 à 8, dans lequel le capteur comprend en outre :
- un capteur configuré pour capturer un signal brut de la partie du cortex préfrontal ;
- un processeur configuré pour traiter le signal brut pour obtenir le signal électrophysiologique, de préférence comprenant un numériseur pour numériser le signal brut en le signal électrophysiologique ;
de préférence le numériseur comprend :
- un amplificateur configuré pour amplifier le signal brut ;
- un convertisseur A/N configuré pour faire subir une conversion A/N au signal brut amplifié.

10. Dispositif selon l'une quelconque des revendications 6 à 9, dans lequel l'activateur comprend un stimulateur ; de préférence l'un des suivants :
- un stimulateur optogénétique ;
- un stimulateur électrique direct ou transdermique ;
- un stimulateur magnétique transcrânien ;
- un stimulateur à ultrasons transcrânien ;
permettant d'atteindre une réduction ou une augmentation de la peur et/ou de l'anxiété lorsque le stimulateur active les PV+ In.

11. Dispositif ou système selon la revendication 10, dans lequel l'activateur comprend en outre un générateur d'impulsions pour générer un signal d'impulsion pour le stimulateur, lorsque le détecteur de phase détecte une phase ascendante des oscillations de 2 à 7 Hz du signal électrophysiologique ou lorsque le détecteur de phase détecte une phase descendante des oscillations de 2 à 7 Hz du signal électrophysiologique.
